# NEUE EUROPÄISCHE PATENTSCHRIFT

(11) **EP 0 347 372 B2**
(45) Veröffentlichungstag und Bekanntmachung des Hinweises auf die Entscheidung über den Einspruch: **27.11.1996**
(45) Hinweis auf die Patenterteilung: 21.07.1993
(21) Anmeldenummer: 89810384.1
(22) Anmeldetag: 24.05.1989
(51) Int. Cl.: A61B 3/10, B25J 19/00

(54) **Höhenverstellbare Tragvorrichtung für ein Instrument**
Instrument holder adjustable in height
Support pour instrument réglable en hauteur

(30) Priorität: 16.06.1988 CH 2333/88
(43) Veröffentlichungstag der Anmeldung: 20.12.1989
(73) Patentinhaber: HAAG-STREIT AG Werkstätten für Präzisionsmechanik, CH-3098 Köniz (CH)
(72) Erfinder: Papritz, Franz, CH-3145 Niederscherli (CH); Widmer, Hansruedi, CH-3145 Niederscherli (CH)
(74) Vertreter: Steiner, Martin

(56) Entgegenhaltungen:
- EP-A- 0 105 076
- EP-A- 0 143 134
- DD-A- 109 729
- DD-A- 257 473
- DE-A- 2 307 044
- DE-A- 3 026 379
- DE-A- 3 031 463
- DE-A- 3 416 823
- DE-A- 3 444 313
- DE-A- 3 511 907
- DE-A- 3 627 340
- DE-A- 3 908 682
- DE-B- 2 717 772
- DE-U- 8 512 337
- FR-A- 2 382 643
- US-A- 3 463 579
- US-A- 3 612 462
- Max Pollermann, "Bauelemente der physikalischen Technik", Springer Verlag 1955, S. 74/75
- H.J. Knab, "Übersicht über Kinematik/Getriebelehre", Nürnberg 1930, S. 96/97

## Beschreibung

Die vorliegende Erfindung betrifft eine höhenverstellbare Tragvorrichtung für ein Instrument, insbesondere ophthalmologisches Instrument, nach dem Oberbegriff des Anspruchs 1. Bekannte Vorrichtungen dieser Art sind mit einer Kompensationsfeder ausgerüstet, welche auf ein bestimmtes Gewicht abgestimmt ist (US-A-3 463 579). In vielen Fällen ist jedoch das zu kompensierende Gewicht nicht ein für alle Mal vorgegeben, indem entweder verschiedenartige Instrumente verwendet werden oder aber Instrumente einer bestimmten Grundausführung mit verschiedenen Zusätzen kombiniert werden können. So kann beispielsweise das Mikroskop einer Spaltlampe mit verschiedenen zusätzlichen Komponenten, beispielsweise einem Tonometer, einem Stereowinkel-Wechsler, einem Strahlenteiler, einem Mitbeobachtertubus und weiteren Aggregaten ergänzt werden. Eine einzige Kompensationsfeder kann jedoch immer nur ein bestimmtes Gewicht befriedigend kompensieren, und eine schlechte Gewichtskompensation erschwert die Vertikalverstellung der Tragvorrichtung bzw. des darauf befindlichen Instrumentes.

Eine höhenverstellbare Tragvorrichtung gemäss Oberbegriff des Anspruchs 1 ist bekannt in Form der Zeissinstrumentenbasis 300 706.

Der vorliegenden Erfindung liegt die Aufgabe zugrunde, die Kompensationskraft auf einfache Weise abstufen zu können, um verschiedene Instrumentengewichte optimal kompensieren zu können. Die erfindungsgemässe Lösung dieser Aufgabe ist im Anspruch 1 beschrieben. Die Zahl und Kraft der Kompensationsfedern kann somit variieren, um eine den Gegebenheiten angepasste Abstufung der gesamten Kompensationswirkung zu erzielen. Vorzugsweise wird jede Kompensationsfeder von einem Kupplungsanker, z.B. einer Kupplungsschraube, durchsetzt, die mit der Basis gekuppelt, z.B. verschraubt werden kann. Das Zu- und Ausschalten bzw. Kuppen und Entkuppeln der zusätzlichen Kompensationsfedern kann daher auch in sehr einfacher Weise, beispielsweise mittels eines Schraubenziehers erfolgen. Es ist aber auch möglich, Mittel zum Kuppen und Entkuppeln vorzusehen, die ohne Werkzeug betätigbar sind.

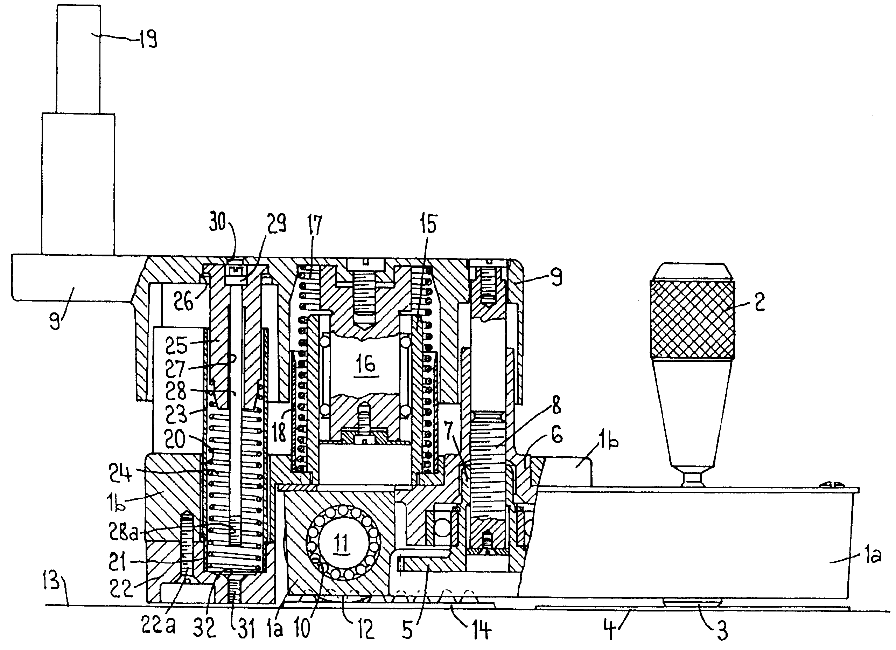

Die Erfindung wird nun anhand eines Ausführungsbeispiels näher erläutert. Die Zeichnung zeigt dieses Ausführungsbeispiel im Vertikalschnitt, teilweise in Ansicht.

Die Tragvorrichtung weist eine Basis 1 aus Teilen 1a und 1b auf. Im Teil 1a ist ein Feineinstellhebel 2 schwenkbar und drehbar gelagert. Das untere Ende dieses Hebels greift in eine Platte 3 die auf einem Gleitbelag 4 abgestützt ist. Durch Schwenken des Hebels 2 kann die Basis 1 gegenüber der abgestützten Platte 3 in der Horizontalebene fein eingestellt werden. Eine Drehung des Hebels 2 um seine Achse wird auf ein Zahnrad 5 übertragen, welches in einer mit dem Teil 1b fest verbundenen Lagerbüchse 6 gelagert ist und aus einem Stück mit einer Spindelmutter 7 besteht. In diese Spindelmutter 7 greift eine Spindel 8, die in der Lagerbüchse 6 vertikal verschiebbar geführt ist, und deren oberes Ende mit einem vertikal beweglichen Oberteil 9 der Tragvorrichtung verbunden ist. Jede Drehung des Hebels 2 wird also über das Zahnrad 5 auf die Spindelmutter 7 übertragen und bewirkt über die Spindel 8 eine vertikale Feinverstellung des Oberteils 9. Eine derartige Feineinstellvorrichtung ist in der oben erwähnten US-A-3 463 579 beschrieben und bedarf keiner weiteren Erläuterung.

In einer Querborung 10 des Teils 1a ist mittels einer Kugelführung eine Welle 11 gelagert. An beiden aus dem Teil 1a vorragenden Enden dieser Welle 11 sind Stützräder 12 befestigt, deren gelochte Felgen je in eine auf dem Instrumententisch 13 befestigte Zahnschiene 14 eingreifen. Die beiden Räder 12 und die Platte 3 ergeben somit eine Dreipunktauflage für die Basis 1. Die Basis kann horizontal in einem bestimmten Bereich grob eingestellt werden, wobei die Platte 3 auf dem Belag 4 gleitet, die Räder 12 auf den Zahnschienen 14 abrollen und damit die Basis 1 in einer bestimmten Orientierung halten, und indem die Basis aufder Welle 11 quer verschoben werden kann.

Im Teil 1b der Basis 1 ist eine Lagerbüchse 15 befestigt, in welcher ein Führungszapfen 16 mittels einer Kugelführung vertikal geführt ist. Der Zapfen 16 ist fest mit dem Oberteil 9 verbunden und dient damit der präzisen Vertikalführung dieses Oberteils. Die Lagerbüchse 15 und der Führungszapfen 16 sind umgeben von einer Druckfeder 17 die zwischen der Basis 1 und dem Oberteil 9 wirkt und damit der Gewichtskompensation der vertikal beweglichen Teile der Tragvorrichtung und eines aufgesetzten Instrumentes dient. Die Kompensationsfeder 17 ist in ihrem unteren Teil in einer Büchse 18 geführt. Die Zeichnung zeigt in Ansicht einen mit dem Oberteil 9 fest verbundenen Lagerzapfen 19 auf dessen Unterteil beispielsweise der Schwenkarm eines Mikroskops einer Spaltlampe und auf dessen Oberteil die Beleuchtungseinheit der Spaltlampe schwenkbar montiert sein kann.

In einer oder mehreren Bohrungen 20 des Teils 1b der Basis 1 bzw. in Bohrungen 21 einer damit mittels einer oder mehreren Schrauben 22a lösbar verbundenen Platte 22 ist eine Hülse 23 angeordnet in der sich eine zusätzliche Kompensationsfeder 24 befindet. Diese zusätzliche Kompensationsfeder ist unten am Grund der Bohrung 21 abgestützt und wirkt oben auf einen in der Hülse 23 vertikal verschiebbaren Kolben 25, dessen oberes Ende lose in eine Ausnehmung 26 des Oberteils 9 eingreift. In einer durchgehenden Bohrung 27 des Kolbens 25 befindet sich eine Schraube 28 deren Kopf 29 in einem erweiterten oberen Teil der Bohrung 27 abgestützt ist. Die Schraube 28 ist lose, axial beweglich und drehbar in die Bohrung 27 eingesetzt. Ihr Schraubenschlitz ist durch eine Oeffnung 30 im Oberteil 9 zugänglich. Die Vorrichtung ist in einer mittleren Stellung dargestellt, d.h. der Oberteil 9 befindet sich weder in seiner obersten noch in seiner untersten Stellung. Das untere, mit Gewinde versehene Ende 28a der Schraube 28 befindet sich hierbei in einem gewissen Abstand über einer koaxial zur Schraube 28 liegenden Gewindebohrung 31 in der Platte 22. Ueber der Gewindebohrung 31 befindet sich ein Führungskonus 32 welcher dazu dient, das mit Gewinde versehene untere Ende 28a der Schraube 28 koaxial über die Gewindebohrung 31 zu lenken, wenn die Schraube bis zur Gewindebohrung abgesenkt wird. Während in der Zeichnung nureine zusätzliche Kompensationsfeder 24 mit den zugehörigen Organen dargestellt ist, können mehrere derartige Federn entweder in einer Reihe nebeneinander in einem Ring oder in einem Mehreck angeordnet sein, um verschiedene Abstufungen der Kompensationskraft zu ermöglichen. Es können dabei auch Kompensationsfedern mit unterschiedlicher Federkraftvorgesehen sein. Bei mit der Platte 22 verschraubten Schrauben 28 kann die Platte 22 mitsamt der oder den Hülsen 23, der oder den Federn 24 und dem oder den Kolben 25 vom Basisteil 1 durch Lösen der Schraube oder Schrauben 22a getrennt und ausgebaut werden, und es kann ein anderes gleichartiges Aggregat mit einer Federodereinem Satzvon Federn eingesetzt werden, die anders bemessen sind.

Beim dargestellten wirksamen Zustand wirkt die zusätzliche Kompensationsfeder 24 über den Kolben 25 auf den Oberteil 9 und unterstützt die Kompensationswirkung der Feder 17. Es kann also hierbei das Gewicht des mit einem bestimmten Zusatzaggregat versehenen Instrumentes optimal kompensiert sein. Wird nun dieses Zusatzaggregat entfernt, soll auch die Kompensationsfederkraft reduziert werden. Das geschieht dadurch, dass das Oberteil 9 vorerst durch Drehung des Hebels 2 in seine untere Endstellung oder mindestens annähernd in diese Endstellung gebracht wird. Bei dieser Stellung liegt das untere, mit Gewinde versehene Ende 28a der Schraube 28 bereits an der Gewindebohrung 31 auf und der Schraubenkopf 29 ist etwas von seiner Auflageschulter in der Bohrung 27 des Kolbens 25 angehoben. Die Oeffnung 30 im Oberteil 9 ist so bemessen, dass mindestens der mit dem Schraubenschlitz versehen oberste Teil der Schraube 28 in diese Oeffnung eintreten kann. Mittels eines Schraubenziehers wird nun die Schraube 28 mit der Gewindebohrung 31 verschraubt, bis sie in dieser Gewindebohrung festsitzt. Der Kolben 25 wird dabei unter Ueberwindung der Kraft der Kompensationsfeder 24 etwas nach unten geschraubt und vom Oberteil 9 entfernt. Die zusätzliche Kompensationsfeder 24 wird damit für alle Vertikalstellungen des Oberteils 9 unwirksam. Soll die zusätzliche Kompensationsfeder wieder wirksam werden, wird der Oberteil 9 mindestens annähernd in seine untere Endstellung gebracht, worauf mittels eines Schraubenziehers die Schraube 28 aus der Gewindebohrung 31 geschraubt wird. Damit wird der Kolben 25 freigegeben und er liegt wieder mit der Kraft der zusätzlichen Kompensationsfeder 24 am Oberteil 9 an und trägt zur Gewichtskompensation bei. Sind mehrere zusätzliche Kompensationsfedern vorhanden können sie wahlweise in der soeben beschriebenen Weise wirksam oder unwirksam gemacht werden.

Ist nicht von vorneherein bekannt, wieviele und/oder welche zusätzliche Kompensationsfedern erforderlich sind, wird vorzugsweise so vorgegangen, dass zuerst alle vorhandenen zusätzlichen Kompensationsfedern wirksam gemacht werden. Sodann wird geprüft, ob das von Hand nach oben gedrückte Oberteil 9 bzw. ein darauf befindliches Instrument nach dem Loslassen um das Spiel in der Mechanik nach unten zurückfedert. In der Regel, d.h. wenn die Belastung nicht ohnehin maximal ist wird dies nicht der Fall sein. Es werden nun zusätzliche Kompensationsfedern ausgeschaltet, bis erstmals das oben erwähnte Zurückfedern nach unten stattfindet womit die richtige optimale Einstellung erreicht ist. Es wird also stets eine nicht ganz vollständige Kompensation angestrebt, damit jedes Spiel in der Vertikaleinstellung ausgeschaltet ist.

Es könnten andere Mittel zur Verankerung des Kolbens 25 an der Basis 1 vorgesehen sein. Anstelle der Schraube 28 die in eine Gewindebohrung 31 eingeschraubt werden kann, könnte eine ähnliche Stange am unteren Ende mit einem quer zur Längsachse der Stange vorstehenden Stift vorgesehen sein, der in der Art eines Bajonettverschlusses durch Drehung in einer Richtung in der Platte 22 verriegelt und durch Drehung in der anderen Richtung entriegelt werden kann. Es könnten auch Mittel vorgesehen sein, um den Kolben 25 an der ihn umgebenden Hülse 23 zu verriegeln. So könnte beispielsweise an diesem Kolben ein radial vorstehender Stift angebracht sein, welcher bei wirksamer zusätzlicher Kompensationsfeder in einem vertikalen Schlitz der Hülse 23 läuft und der am unteren Ende dieses vertikalen Schlitzes durch Verdrehung des Kolbens von oben in einen in Umfangsrichtung verlaufenden Teil des Schlitzes eingeführt werden kann. Der Kolben könnte damit jeweils durch leichte Drehung mit der zugeordneten Hülse 23 gekuppelt werden, um die entsprechende zusätzliche Kompensationsfeder umwirksam zu machen. Anstelle des Kolbens 25 könnte auch ein Teller vorgesehen sein, über welchen die zusätzliche Kompensationsfeder 24 auf das Oberteil 9 wirkt und in welchem die Schraube 31 oder ein entsprechendes Kupplungsorgan aufgehängt ist. Es könnten auch andere, im Basisteil 1b dauernd verankerte Kupplungsorgane vorgesehen sein, die von oben eingerückt werden können, um den Kolben 25 mit dem Basisteil 1b zu kuppeln und damit die Kompensationsfeder 24 unwirksam zu machen.

Beim Ausführungsbeispiel ist aus praktischen Gründen die Kupplungsschraube 28 von oben zugänglich und dient der Verankerung des Kolbens 25 durch Verschraubung mit der Basis. Es könnte aber auch umgekehrt eine von unten betätigbare Kupplungsschraube mit dem Kolben verschraubt werden.

Die Feder oder Federn brauchen auch nicht unbedingt in der Bewegungsachse des zu kompensierenden Teils wirken. Es könnten also z.B. horizontal liegende Federn über geeignete Getriebe aufvertikal verstellbare Teile wirken, in welchem Falle es durchaus vorteilhafter sein könnte, das gegenseitige Kuppeln und Entkuppeln der Federenden von der Seite der ortsfesten Abstützung der Feder oder Federn her vorzunehmen. Die Mittel zum Kuppen bzw. Entkuppeln der Federenden können aber auch von einer beliebigen Seite her von Hand oder mittels Werkzeugen betätigbar sein, wobei z.B. ein drehbarer Kupplungsanker nötigenfalls über ein Winkelgetriebe betätigt werden kann.

## Patentansprüche

1. Höhenverstellbare Tragvorrichtung mit einem Basisteil (1a, 1b) und einem höhenverstellbaren Oberteil (9), an dem ein Instrument, insbesondere ophthalmologisches Instrument, montierbar ist, mit einer Kompensationsfeder (17) zwischen Basisteil und Oberteil zur Kompensation des Gewichts des Instrumentes und des höhenverstellbaren Oberteils durch Federkraft, wobei mindestens eine zusätzliche Kompensationsfeder (24) zwischen Basisteil und Oberteil angeordnet ist, und wobei an jeweils einem Ende der zusätzlichen Kompensationsfeder(n) Mittel (25, 28, 29, 31,) vorgesehen sind, um die zusätzliche(n) Kompensationsfeder(n) wahlweise wirksam oder unwirksam zu machen, dadurch gekennzeichnet, dass das auf das höhenverstellbare Oberteil (9) wirkende Ende der zusätzlichen Kompensationsfeder(n) (24) durch die Mittel (25, 28, 29, 31) mit dem Basisteil (1) verbindbar ist, um sie unwirksam zu machen bzw. vom Basisteil lösbar ist, um sie wirksam zu machen.

2. Vorrichtung nach Anspruch 1, dadurch gekennzeichnet, dass die zusätzliche Kompensationsfeder (24) von einem Kupplungsanker, z. B. einer Kupplungsschraube (28) durchsetzt ist, die mit der Basis (1, 22) kuppelbar, z. B. verschraubbar ist.

3. Vorrichtung nach Anspruch 1 oder 2, dadurch gekennzeichnet, dass die zusätzliche Kompensationsfeder (24) über einen Kolben (25) oder Teller auf das höhenverstellbare Oberteil (9) wirkt.

4. Vorrichtung nach Anspruch 3, dadurch gekennzeichnet, dass der Kupplungsanker, z. B. die Kupplungsschraube (28), im Kolben (25) oder Teller lose aufgehängt ist.

5. Vorrichtung nach Anspruch 3 oder 4, dadurch gekennzeichnet, dass der Kolben (25) oder Teller und die zusätzliche Kompensationsfeder (24) in einer Führungshülse (23) geführt sind.

6. Vorrichtung nach einem der Ansprüche 1 bis 5, dadurch gekennzeichnet, dass ein durch Drehung in der Art eines Bajonettverschlusses kuppelbarer und entkuppelbarer Kupplungsanker, Kolben oder Teller vorgesehen ist, oder dass umstellbare Verriegelungsmittel für den Kolben, den Teller oder direkt die zusätzliche Kompensationsfeder vorgesehen sind.

7. Vorrichtung nach einem der Ansprüche 1 bis 6, dadurch gekennzeichnet, dass die zusätzlichen Kompensationsfedern (24) unterschiedliche Federkräfte aufweisen.

8. Vorrichtung nach einem der Ansprüche 1 bis 7, dadurch gekennzeichnet, dass die zusätzlichen Kompensationsfeder(n) (24) auf einer Platte (22) abgestützt ist bzw. sind, die lösbar mit dem Basisteil (1) verbunden, z. B. verschraubt (22a) ist.

## Claims

1. Height adjustable instrument holder, comprising a base (1a, 1b) and an upper part (9) whose height is adjustable and to which an instrument, more particularly an ophthalmological instrument, can be fastened, a compensation spring (17) being provided between said base and said upper part in order to compensate the weight of said instrument and of said height adjustable upper part by spring force, at least one auxiliary compensation spring (24) being arranged between said base and said upper part, and means (25, 28, 29, 31) being provided at one of the ends of each auxiliary compensation spring in order to selectively render said auxiliary compensation spring(s) effective or ineffective, characterized in that the end(s) of said auxiliary compensation spring(s) (24) acting upon the height-adjustable upper part (9) are capable of being connected, by said means (25, 28, 29, 31), to said base (1) in order to render them ineffective, or disconnected from said base in order to render them effective.

2. Holder according to claim 1, characterized in that the auxiliary compensation spring (24) is traversed by a coupling anchor, e.g. a coupling screw (28) which can be coupled with the base (1, 22), e.g. by screwing.

3. Holder according to claim 1 or 2, characterized in that the auxiliary compensation spring (24) acts by a piston (25) or plate on the upper part (9) adjustable in height.

4. Holder according to claim 3, characterized in that the coupling anchor e.g. the coupling screw (28) hangs up freely in the piston (25) or the plate.

5. Holder according to claim 3, characterized in that the piston (25) or the plate and the auxiliary compensation spring (24) are guided in a guiding bushing (23).

6. Holder according to one of claims 1 to 5, characterized in that there are provided a coupling anchor, a piston or plate capable to be coupled, resp. uncoupled by rotation according to the principle of a bayonet coupling or adjustable locking means for the piston, the plate or directly the auxiliary compensation spring.

7. Holder according to one of claims 1 to 6, characterized in that the auxiliary compensation spring(s) (24) are of different spring forces.

8. Holder according to one of claims 1 to 7, characterized in that the auxiliary compensation spring(s) (24) are supported, resp. lie on a plate (22) which is detachably connected with the base (1), e.g. by screwing.

## Revendications

1. Support réglable en hauteur, avec une base (1a, 1b) et une partie supérieure (9) réglable en hauteur, à laquelle un instrument, plus particulièrement instrument ophthalmologique, est susceptible d'être monté, avec un ressort de compensation (17) entre la base et la partie supérieure pour la compensation, par la force d'un ressort, du poids de l'instrument et de la partie supérieure réglable en hauteur, au moins un ressort de compensation auxiliaire (24) étant disposé entre base et partie supérieure, et des moyens (25, 28, 29, 31) étant prévus à l'une des extrémités du ou des ressort(s) de compensation auxiliaire(s) pour rendre au choix effectifs ou ineffectifs les ressort(s) de compensation auxiliaire(s), caractérisé en ce que l'extrémité du ou des ressort(s) de compensation auxiliaire(s) agissant sur la partie supérieure (9) réglable en hauteur est capable d'être reliée, par lesdits moyens (25, 28, 29, 31), à la base (1) pour les rendre ineffectifs, ou découplée pour les rendre effectifs.

2. Support selon la revendication 1, caractérisé en ce que le ressort de compensation auxiliaire (24) est traversé par une ancre de couplage, p.ex. une vis de couplage (28) qui peut être couplée avec la base (1, 22), p.ex. par vissage.

3. Support selon la revendication 1 ou 2, caractérisé en ce que le ressort de compensation auxiliaire (24) agit par un piston (25) ou assiette sur la partie supérieure (9) réglable en hauteur.

4. Support selon la revendication 3, caractérisé en ce que l'ancre de couplage, p.ex. la vis de couplage (28) est suspendue librement dans le piston (25) ou l'assiette.

5. Support selon la revendication 3, caractérisé en ce que le piston (25) ou l'assiette et le ressort auxiliaire de compensation (24) sont guidés dans une douille de guidage (23).

6. Support selon l'une des revendications 1 à 5, caractérisé en ce que sont prévus une ancre de couplage, un piston ou assiette susceptible d'être couplé, resp. découplé par rotation selon le principe d'une fermeture à bajonnette, ou des moyens de verrouillage réglables pour le piston, l'assiette ou directement le ressort de compensation auxiliaire.

7. Support selon l'une des revendications 1 à 6, caractérisé en ce que les ressorts de compensation auxiliaires (24) ont des forces de ressort différentes.

8. Support selon l'une des revendications 1 à 7, caractérisé en ce que les ressort(s) de compensation auxiliaire(s) (24) reposent, resp. sont sur une plaque (22) qui est liée de manière détachable avec la base (1), p.ex. par vissage (22a).
